(19) Europäisches Patentamt
European Patent Office
Office européen des brevets

(11) EP 1 340 489 A1

(12) **EUROPEAN PATENT APPLICATION**

(43) Date of publication:
**03.09.2003 Bulletin 2003/36**

(51) Int Cl.[7]: **A61K 7/13**

(21) Application number: **02004307.1**

(22) Date of filing: **28.02.2002**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Kao Chemicals Europe S.L.**
**08210 Barbera del Valles, Barcelona (ES)**

(72) Inventors:
- **Denzer, Horst**
**46446 Emmerich (DE)**
- **Hamke, Meijer**
**46446 Emmerich (DE)**
- **van der Veen, Reinout**
**46446 Emmerich (DE)**

(74) Representative: **HOFFMANN - EITLE**
**Patent- und Rechtsanwälte**
**Arabellastrasse 4**
**81925 München (DE)**

(54) **Compositions comprising levelling agent for dyeing keratinous fibres, use and method**

(57) New dyeing compositions, containing improved levelling agents, for dyeing of keratinous fibres, especially human hair, making more uniform the colour of the dyed fibres, are. provided. The compositions contain one or more compounds of the following formulae (I) and (II):

I 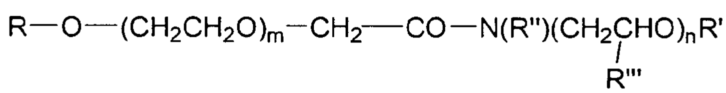

II 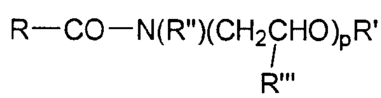

wherein
R is an alkyl group or alkenyl group having 6 to 24 carbon atoms;
R', R'' and R''' are independently a hydrogen atom or an alkyl group having 1 to 5 carbon atoms which is optionally hydroxylated;
m has a value in the range of 4 to 10;
n has a value in the range of 0 to 10; and
p has a value in the range of 1 to 10, with the proviso that when p = 1, at least one of R', R'' and R''' is not a hydrogen atom.

EP 1 340 489 A1

## Description

### Technical field

The present invention relates to dyeing compositions containing improved levelling agents.

### Prior Art

For centuries, preparations to dye keratinous fibres have been commonly used, i.e. preparations to dye human keratinous fibres, as human hair, are among the oldest cosmetics known. Their use can be traced back to the ancient Egyptians, Greeks and Romans. The earliest hair colourings were vegetable in origin and included henna, indigo, saffron, camomile, nutgalls and many others.

Hair dyes can be classified by the dye to be used therefor, or whether they have bleaching action of melanin or not. Typical examples include a two-part permanent hair dye composed of a first part containing an alkali agent, an oxidation dye and a direct dye such as nitro dye and a second part containing an oxidizing agent; and one-part semipermanent hair dye containing an organic acid or an alkali agent, and a direct dye such as acid dye, basic dye or nitro dye.

The preparations to dye keratinous fibres often contain various reagents in addition to dyes. The purpose of these reagents is, in many cases, to control the manner in which dyes are adsorbed on the fibres and hence to control the evenness of the final dyeing, to produce a uniform distribution of the dyestuff.

These compounds, collectively referred to as "levelling agents", may be inorganic salts such as sodium sulphate, although various types of surfactants were found to be more effective as levelling agents. Anionic, cationic or amphoteric surfactants are commonly used, either alone or in blends, for dyeing both human and animal keratinous fibres. The mode of action of these levelling agents, especially the surfactants, usually involves formation of a complex with the dye. The complex is adsorbed more evenly by the fibres than the dye alone.

Improved evenness of dye adsorption is particularly important when dyeing damaged fibres in order to avoid an undesired appearance in the final dyed fibres, and thus surfactant levelling agents are generally employed when dyeing damaged fibres. The damage to the fibres can be produced by repeated dyeing to such an extent that the tip and root portions of fibres have markedly different dyeing properties. The above-mentioned undesired appearance refers to an undesired speckled effect arising from differences in colour between adjacent fibres or portions of the same, an effect often associated with damaged keratinous fibres.

It would prefer, that he above-mentioned requirement of producing an uniform distribution of the dyestuff would be achieved without losing the intensity of hair colouring, obtaining optimal colour results (adequate colouring of some type of keratinous fibres).

Commonly, alkoxylated fatty amines and their quaternary derivatives have been used as levelling agents for dyeing keratinous fibres, especially for dyeing keratinous animal fibres, as disclosed in US 4 778 919, US 5 443 598 and US 6 086 638.

US4 168 145 describes a process for the dyeing of the leather side of wool- and fur-bearing skins, wherein the wool- or fur-bearing skins are dyed in aqueous medium with (a) a hydrosoluble, sulpho group-containing sulphur dye or a mixture of such dyes in the presence of (b) a dye-substantive uptake assistant or a mixture of such assistants and (c) a non-ionic or anionic hydrophilic dispersant or a mixture of such dispersants. It is indicated that particular categories of suitable non-ionic emulsifiers are oxyethylation products of fatty acids, fatty amides, fatty alcohols or mono- or dialkylphenols or of mono- or difatty acid esters of sorbitol, which may also contain propyleneoxy units. The fatty radicals contain preferably 9-24, more preferably 12-22 carbon atoms and the corresponding hydrocarbon radicals may be conventional alkyl and/or alkenyl radicals. The fatty acid amides are preferably amides of lauric, palmitic, myristic, oleic, stearic, arachidic and behenic acid and technical mixtures such as coconut fatty acid and tallow fatty acid. The degree of oxyethylation for the non-ionic dispersant (c) is advantageously in the range of 3-70, preferably in the range of 3-50, more preferably 4-30. However, in the examples it is not provided any specific description of these fatty amides.

The international patent application WO-A-9534273 describes aqueous hair dye formulations containing a direct dye, a cationic polymer, an $\alpha$-hydroxyacid or alkali metal or ammonium salt thereof, and an ether carboxylic acid of formula $R^1$-O-$(CH_2CH_2O)_nCH_2COOH$ or alkali metal or ammonium salt thereof. In the description it is stated that further ingredients could be used, i.e. fatty acid alkanolamides as emulsifiers or moisturizers. However, in the examples it is not provided any specific description of these fatty acid alkanolamides.

US 6 312 677 describes cosmetic compositions based on nonionic surfactants and cationic or amphoteric substantive polymers and its use as a dyeing or bleaching vehicle. The nonionic surfactants are chosen from oxyethylenated and/or oxypropylenated and/or polyglycerolated fatty alcohols which are linear or branched. In the description it is stated that the compositions could contain further ingredients, as adjuvants such as alkalinizing agents, preservatives, sequestering agents, perfumes, sunscreen agents, fatty amides, natural or synthetic sterols, $C_{10}$-$C_{18}$ fatty acids and polymers. The fatty amides are especially chosen from oleic or lauric diethanolamide, coconut mono- or diethanolamide, and oxyethylenated ($C_{13}$ -$C_{15}$) alkyl ether carboxylic acid monoethanolamide containing 2 mol of ethylene oxide. However, in the examples it is not provided any specific description of these fatty amides.

It is clear that the problem of obtaining dyeing compositions containing improved levelling agents that render dyeing of keratinous fibres, making the colour of the dyed fibres more uniform, still requires new solutions to obtain easier and cheaper compositions with a higher effectiveness.

**Summary of the invention**

The present invention provides dyeing compositions containing improved levelling agents that render dyeing of keratinous fibres, especially human hair, making more uniform the colour of the dyed fibres.

The present invention also provides a method for dyeing keratinous fibres with the dyeing compositions object of the invention containing the improved levelling agents.

The present invention also provides the use of specific amides as levelling agents in compositions for dyeing of keratinous fibres, especially human hair.

**Description of the invention**

The dyeing composition of the present invention comprises

a) a dyestuff; and

b) one or more compounds of the following formulae (I) and (II):

I 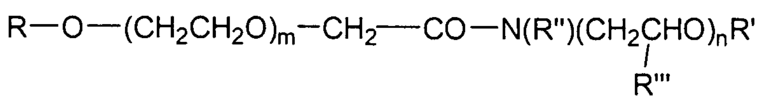

II 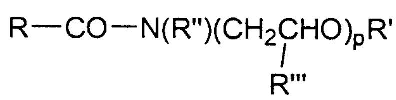

wherein

R is an alkyl group or alkenyl group having 6 to 24 carbon atoms;

R', R'' and R' ' ' are independently a hydrogen atom or an alkyl group having 1 to 5 carbon atoms which is optionally hydroxylated;

m has a value in the range of 4 to 10;

n has a value in the range of 0 to 10; and

p has a value in the range of 1 to 10, with the proviso that when p = 1, at least one of R', R'' and R' ' ' is not a hydrogen atom.

The dyestuff can be a direct dye or a combination therefor. Examples of direct dyes include Basic Blue 7 (C. I. 42595), Basic Blue 26 (C.I. 44045), Basic Blue 99 (C.I. 56059), Basic Violet 10 (C.I. 45170), Basic Violet 14 (C.I. 42515), Basic Brown 16(C.I. 12250), Basic Brown 17 (C.I. 12251), Basic Red 2 (C.I. 50240), Basic Red 22 (C.I. 11055), Basic Red 76 (C.I. 12245), Basic Red 118 (C.I. 12251:1) and Basic Yellow 57(C.I. 12719); and basic dyes as described in Japanese Patent Publication No. Sho 58-2204, Japanese Patent Application Laid-Open No. Hei 9-118832, Japanese Language Laid-Open Publication (PCT) No. Hei 8-501322 or Japanese Language Laid-Open Publication (PCT) No. Hei 8-507545. If a single direct dye is used, it is preferably added in an amount of 0.01 to 20 wt.-%, more preferably 0.05 to 10 wt. -%, especially 0.1 to 5 wt.-% on the basis of the entirety of the composition (after mixing of all the parts when a two-part or three-part composition is employed; this will apply equally hereinafter). When another direct dye is used in combination, the content of it with the first direct dye preferably ranges from 0.05 to 10 wt.-%, especially 0.1 to 5 wt.-%, based on the whole composition.

In the above formulae I and II, R represents an alkyl or alkenyl group, having 6 to 24 carbon atoms, preferably 12 to

24 carbon atoms, and with respect to formula (I), 12 to 18 carbon atoms are most preferred. It is particularly preferable that R is derived from natural fat and oil as well as synthetic triglycerides. Preferred fats and oils include palm oil, coconut oil, sunflower oil, rapeseed oil, castor oil, olive oil, soybean oil; and animal fat such as tallow, bone oil; fish oil, hardened oils and semihardened oils thereof; and mixtures thereof. Particularly preferred are alkyl or alkenyl groups derived from coconut oil, palm oil, rapessed oil and tallow such as beef tallow.

R', R ' ' and R' ' ' in the above formulae represent independently from one another a hydrogen atom or an alkyl group having 1 to 5 carbon atoms which may optionally be hydroxylated. This includes compounds wherein in formulae I and II R' ' ' represents both a hydrogen atom and an alkyl group in the event that the compound contains more than one alkylene oxide unit (n>1; p>1), i.e. different alkylene oxide units may be present within one molecule. R', R' ' and R' ' ' preferably represent a hydrogen atom, a methyl group, an ethyl group, or a hydroxyethyl group. Most preferred for R' is a hydrogen atom, for R ' ' and R ' ' ' a hydrogen atom or a methyl group.

Preferred values for m of formula I are in the range of 4 to 8.

Preferred values for n of formula I are in the range of 0 to 5, more preferred are values in the range of 0 to 2, most preferred is a value of 1.

The sum of m and n in formula I has preferably a value in the range of 5 to 9.

Particularly preferred compounds of formula I are those wherein m has a value in the range of 4 to 8, n has a value of 1, R is an alkyl group or alkenyl group having 12 to 18 carbon atoms, more preferably 12 to 14 carbon atoms; R' and R' ' represent a hydrogen atom, and R' ' ' represents a hydrogen atom or a methyl group. These include Polyoxyethylene (4.5) ($C_{12}$-$C_{14}$) alkyl ether carboxylic acid monoethanolamide and Polyoxyethylene (7) ($C_{12}$-$C_{14}$) alkyl ether carboxylic acid monoethanolamide.

In formula II, p has preferably a value in the range of 3 to 8, more preferably 3 to 5, most preferably a value of 3 or 4, particularly if R ' ' represents a hydrogen atom.

Particularly preferred as compounds of the formula (II) is also a composition marketed under the name Aminol N by Kao Chemicals Europe, S.L., which is Polyoxyethylene (4) Rapeseedamide.

Other preferred compounds of formula II are those wherein p has a value of 1 or 2, and R' ' represents a methyl group, ethyl group, or hydroxyethyl group. In this embodiment, R is preferably derived from palm kernel oil. This includes N-methylethanolamine Palm Kernel Oil Acid Amide.

Particularly preferred compounds of formula II are those satisfying the following formula III:

III
$$R-CO-N(R'')(CH_2\underset{Me}{\underset{|}{C}H}O)_r(CH_2CH_2O)_sR'$$

wherein r has a value in the range of 1 to 3, more preferably 1, s has a value in the range of 1 to 3, and Me means a methyl group, and R' and R' ' preferably represent a hydrogen atom. In particularly preferred compounds of formula III, R is an alkyl or alkenyl group having 12 to 24 carbon atoms, for example those derived from coconut oil or rapeseed oil.

The amount of levelling agent to add to compositions for dyeing keratinous fibres depends on several factors. Generally, the amount of levelling agent is preferably between 0.05wt.-% to 25wt.-%, more preferably between 5 and 20 wt.% with respect to the total weight of the dyeing composition.

The hair dye composition of the present invention is preferably adjusted to pH 6 to 11, with pH 8 to 11 being more preferred. Examples of the alkali agent to be used for pH adjustment includes ordinarily employed ones such as ammonia, organic amines and salts thereof. The alkali agent is preferably added in an amount of 0.01 to 20 wt.-%, more preferably 0.1 to 10 wt.-%, especially 0.5 to 5 wt.-%.

In the hair dye composition of the present invention, ethoxylated products derived from polyhydric alcohols can be incorporated. The degree of ethoxylation of said ethoxylated products is at least 0.5, preferably more than 2. Examples of ethoxylated products derived from polyhydric alcohols can be diethylene glycol, polyethylene glycols (PEG's) and other already ethoxylated polyhydric alcohols. Examples of such products are PEG-300, PEG-1500, and ethoxylated glycerine having an ethoxylation degree in the range of 2 to 20, preferably 3 to 10. Products derived from glycerine are preferred in view with their dermatological properties. The preparation of useful ethoxylated glycerines is disclosed in EP-A-1 045 021.

The amount of ethoxylated products derived from polyhydric alcohols is between 0.5wt.-% to 25wt.-% with respect to the total weight of the dyeing composition.

In the hair dye composition of the present invention, an oxidizing agent can be incorporated. In this case, hair dyeing and bleaching can be carried out simultaneously, which facilitates more vivid hair dyeing. Ordinarily employed oxidizing agents, for example, hydrogen peroxide, persulfates such as ammonium persulfate, potassium persulfate

and sodium persulfate, perborates such as sodium perborate, percarbonates such as sodium percarbonate and bramates such as sodium bromate and potassium bromate are usable. Out of them, hydrogen peroxide is especially preferred. The oxidizing agent is added in an amount of 0.5 to 10 wt.-%, especially 1 to 8 wt.-%, on the basis of the entirety of the composition.

In the hair dye composition of the present invention, an oxidation dye can be incorporated further. This incorporation enables markedly vivid dyeing not attainable by the single use of an oxidation dye. As the oxidizing agent, the above-exemplified oxidizing agents can be used, with hydrogen peroxide being particularly preferred. Alternatively, an oxidizing enzyme such as lactase can be employed. For the oxidation dye, known developers and couplers ordinarily employed for an oxidation type hair dye can be used.

Examples of the developer include p-phenylenediamines having one or several groups selected from NH2-, NHR- and NR2-groups (R represents a C(1-4) alkyl or hydroxyalkyl group) such as p-phenylenediamine, p-toluylenediamine, N-methyl-p-phenylenediamine, chloro-p-phenylenediamine, 2-(2'-hydroxyethylamino)-5-aminotoluene, N,N-bis-(2-hydroxyethyl)-p-phenylenediamine, 2-hydroxyethyl-p-phenylenediamine, 2,6-dimethyl-p-phenylenediamine, methoxy-p-phenylenediamine, 2,6-dichloro-p-phenylenediamine, 2-chloro-6-methyl-p-phenylenediamine, 6-methoxy-3-methyl-p-phenylenediamine, 2,5-diaminoanisole, N-(2-hydroxypropyl)-p-phenylenediamine and N-2-methoxyethyl-p-phenylenediamine; 2,5-diaminopyridine derivatives and 4,5-diaminopyrazole derivatives; p-aminophenols such as p-aminophenol, 2-methyl-4-aminophenol, N-methyl-p-aminophenol, 3-methyl-4-aminophenol, 2,6-dimethyl-4-aminophenol, 3,5-dimethyl-4-aminophenol, 2,3-dimethyl-4-aminophenol and 2,5-dimethyl-4-aminophenol; o-aminophenols, o-phenylenediamines, 4,4'-diaminophenylamine and hydroxypropylbis(N-hydroxyethyl-p-phenylenediamine); and salts thereof.

Examples of the coupler include 1 -naphthol, 1,5-dihydroxynaphthalene, 1,7-dihydroxynaphthalene, 2,7-dihydroxynaphthalene, 5-amino-2-methylphenol, 5-(2'-hydroxyethylamino)-2-methylphenol, 2,4-diaminoanisole, m-toluylenediamine, resorcin, m-phenylenediamine, m-aminophenol, 4-chlororesorcin, 2-methylresorcin, 2,4-diaminophenoxyethanol, 2,6-diaminopyridine, 2-amino-3-hydroxypyridine, 4-hydroxyindole, 6-hydroxyindole, 2,4-diamino-6-hydroxypyrimidine, 2,4,6-triaminopyrimidine, 2-amino-4,6-dihydroxypyrimidine, 4-amino-2,6-dihydroxypyrimidine, 4,6-diamino-2-hydroxypyrimidine and 1,3-bis(2,4-diaminophenoxy)propane; and salts thereof. As a developer or coupler, at least one of the above-exemplified ones can be used. Although no particular limitation is imposed on its content, it is added in an amount of 0.01 to 20 wt.%, especially 0.5 to 10 wt.% based on the whole composition.

To the hair dye composition of the present invention, a known auto-oxidation dye typified by an indole or an indoline, or a known direct dye such as a nitro dye or a disperse dye can also be added. Addition of a polyol, polyol alkyl ether, cationic or amphoteric polymer or silicone to the hair dye composition of the present invention is preferred, because the resulting hair dye composition has improved cosmetic effects.

In addition to the above-described components, those ordinarily employed as a raw material for cosmetics can be added to the hair dye composition of the present invention, within an extent not impairing the advantages of the present invention. Examples of such an optional component include hydrocarbons, animal or vegetable fats and oils, higher fatty acids, organic solvents, penetration promoters, cationic surfactants, natural or synthetic polymers, higher alcohols, ethers, amphoteric surfactants, nonionic surfactants, protein derivatives, amino acids, antiseptics, chelating agents, stabilizing agents, antioxidants, plant extracts, crude drug extracts, vitamins, colorants, perfumes and ultraviolet absorbers.

The composition for dyeing keratinous fibres of the present invention can be prepared in a conventional manner into a one-part composition, a two-part composition having a first-part component containing an alkali agent and a second-part component containing an oxidizing agent, or a third-part composition having, in addition to these two components, a powdery oxidizing agent such as persulfate. The direct dye can be incorporated in either one or both of these components of the two-part or three-part composition. The one-part type is applied to the hair directly, while the two-or three-part type is applied to the keratinous fibres after mixing these parts upon hair dyeing.

No particular limitation is imposed on the form of the composition for dyeing keratinous fibres of the present invention. Examples include powder, transparent liquid, emulsion, cream, gel, paste, aerosol, and aerosol foam. It preferably has a viscosity of 2000 to 100000 mPa-s in the stage of application to the hair (after mixing of all the parts when a two-part or three-part type composition is employed).

A method for dyeing keratinous fibres comprising applying to such fibres, an effective amount of at least one composition of the present invention, is also included in the subject of the present invention.

The use of a compound of formula (I) or (II) as levelling agent in compositions for dyeing keratinous fibres is also included in the subject of the present invention.

The following examples are given in order to provide a person skilled in the art with a sufficiently clear and complete explanation of the present invention, but should not be considered as limiting of the essential aspects of its subject, as set out in the preceding portions of this description.

**Examples**

Preparation of the hair dyes compositions

Compounds employed in the below-described examples are as follows:

Table 1

| Examples | **Levelling agent** |
|---|---|
| 1 | Aminol N |
| 2 | Compound (a) + Glycerin 3 EO (1:1 wt.-%) |
| 3 | Compound (a) + Glycerin 6 EO (1:1 wt.-%) |
| 4 | Compound (b) + Glycerin 6 EO (1:1 wt.-%) |
| 5 | Compound (c) + Glycerin 6 EO (1:1 wt.-%) |
| 6 | Aminol N + Compound (e) (1:1 wt.-%) |
| 7 | Compound (d) |
| 8 | Compound (e) |
| 9 | Compound (f) |
| Comp. I | Coconut Fatty Acid Monoethanolamide (CME) |
| Comp. II | Aminol A-15 |
| Comp. III | Compound (g) |

The levelling agents indicated below are the following:

Aminol N, (95 wt.-% active matter), Polyoxyethylene (4) Rapeseedamide, marketed by Kao Chemicals Europe, S.L.

Aminol A-15, (97 wt.-% active matter), C13-15 Pareth Carboxamide MEA, marketed by Kao Chemicals Europe, S.L.

Compound (a), compound according to formula (II) wherein R', R'' and R''' are H, p=3 and R= Coconut
*$R\text{-}CO\text{-}NH(CH_2CH_2O)_3\text{-}H$ (R= Coconut)*

Compound (b), compound according to formula (II) wherein R' and R' ' are H; and R' ' ' is H and methyl; p=3 and R= Rapeseed Oil; corresponding to a compound of formula (III) wherein R' and R' ' are H; r =1, s=2, and R= Rapeseed Oil.
*$R\text{-}CO\text{-}NHCH_2CH(CH_3)\ O(CH_2CH_2O)_2\text{-}H$ (R= Rapeseed Oil)*

Compound (c), compound according to formula (II) wherein R' and R' ' are H; and R' ' ' is H and methyl; p=3 and R= Coconut; corresponding to a compound of formula (III) wherein R' and R' ' are H; r =1, s=2, and R= Coconut.
*$R\text{-}CO\text{-}NHCH_2CH\ (CH_3)\ O\ (CH_2CH_2O)_2\ \text{-}H$ (R= Coconut)*

Compound (d), compound according to formula (II) wherein R' and R' ' 'are H; R' ' is methyl; p=1 and R= Palm Kernel Oil
*$R\text{-}CO\text{-}N(CH_3)CH_2CH_2OH$ (R= Palm Kernel Oil)*

Compound (e), compound according to formula (I) wherein R', R'' and R''' are H; n=1; m=4.5 and R= $C_{12}$ -$C_{14}$
*$R\text{-}O\text{-}\ (CH_2CH_2O)4.5\text{-}CH_2\text{-}CO\text{-}NH\text{-}CH_2CH_2OH$ (R= $C_{12}$ -$C_{14}$)*
Polyoxyethylene (4.5) ($C_{12}$ -$C_{14}$) alkyl ether carboxylic acid monoethanolamide

Compound (f), compound according to formula (I) wherein R', R' ' and R' ' ' are H; n=1; m=7 and R= $C_{12}$ -$C_{14}$
*$R\text{-}O\text{-}\ (CH_2CH_2O)_7\ \text{-}CH_2\text{-}CO\text{-}NH\text{-}CH_2CH_2OH$ (R= $C_{12}$ -$C_{14}$)*
Polyoxyethylene (7) ($C_{12}$ -$C_{14}$) alkyl ether carboxylic acid monoethanolamide

Compound (g), compound according to formula (I) wherein R', R ' ' and R' ' ' are H; n=1; m=3 and R= $C_{12}$ -$C_{14}$

*R-O- $(CH_2CH_2O)_3$-$CH_2$-CO-NH-$CH_2CH_2OH$ (R= $C_{12}$ -$C_{14}$)*
Polyoxyethylene (3) ($C_{12}$ -$C_{14}$) alkyl ether carboxylic acid monoethanolamide

In a manner known per se in the art, hair dyes compositions as shown in Table 2 were prepared. The levelling agent is selected, in each case, from the ones indicated in table 1. The data appearing in each of the following tables represents weight percentage (wt.-%).

Table 2

| 1st part | Components | Quantity |
|---|---|---|
| | Hydroquinone | 0.15 |
| | 1-Phenyl-3-methyl-5-pyrazolone | 0.15 |
| | Polyoxyethylene (9) Oleyl Ether | 10 |
| | Oleic Acid | 10 |
| | Levelling Agent | 15 |
| | Monoethanolamine | 7.5 |
| | Ethanol | 10 |
| | Toluene-2,5-diamine | 0.61 |
| | 1,3-Benzenediol | 0.55 |
| | Sodium Sulfite | 0.50 |
| | Ascorbic Acid | 0.50 |
| | Tetrasodium ethylendiaminetetraacetate | 0.50 |
| | Deionized water | Balance |

| 2nd part | Components | Quantity |
|---|---|---|
| | 35 wt. % aqueous hydrogen peroxide | 16.29 |
| | Cetyl Alcohol | 2.00 |
| | Glycerin | 1.00 |
| | Quartamin ® D-2345P (product of Kao Corporation, dialkyl dimethyl ammonium chloride, 75 wt. % active matter) | 1.00 |
| | Quartamin ® 86PC (product of Kao Corporation, stearyl trimethyl ammonium chloride, 65 wt. % active matter) | 3.00 |
| | LC-2 ® (product of Kao Corporation, Isostearyl Glyceryl Pentaerythrityl Ether) | 1.00 |
| | Amodimethicone SM8702C (trade name; product of Dow Corning Toray Silicone) | 2.00 |
| | Phosphoric acid | Amount to adjust pH to 3.5 |
| | Deionized water | Balance |

Evaluation of the Levelling Effect

For the preparation of the damaged hair, 1 g of goat tress (white colour) is treated 3 times with a powder bleach product (INAZUMA ® Bleach of Kao Corporation).
For each hair dye composition described before, having one part and second part mixed at a weight ratio of 1:1, 20 g was applied for 30 minutes at 30°C to 2 healthy goat tresses (white colour) and 2 damaged goat tresses. After that, each tress was rinsed, washed with a standard shampoo and then dried with air.
The brightness of hair after the coloration treatment was determined with Colorimeter Computer ND-1010C (supplied by Nippon Denshoku Kogyo K.K.)
The levelling effect (LE) of each levelling agent of table 1 was calculated as follows:

LE = ΔE DH(colour intensity on damaged hair) - ΔE

HH(colour intensity on healthy hair)

Representing ΔE the difference in colour between each tress before and after the treatment with the dyeing composition.
Smaller values of LE indicate the better levelling effect of the levelling agent.
Results are indicated in table 3.

Table 3

| Examples | Levelling Agent | LE | ΔE (HH) |
|---|---|---|---|
| 1 | Aminol N | 9.20 | 38.76 |
| 2 | Compound (-a) + Glycerin 3 EO (1:1 wt.-%) | 10.36 | 38.61 |
| 3 | Compound (a) + Glycerin 6 EO (1:1 wt.-%) | 11.23 | 37.34 |
| 4 | Compound (b) + Glycerin 6 EO (1:1 wt.-%) | 10.52 | 37.87 |
| 5 | Compound (c) + Glycerin 6 EO (1:1 wt.-%) | 11.06 | 37.36 |
| 6 | Aminol N + Compound (e) (1:1 wt.-%) | 10.33 | 37.80 |
| 7 | Compound (d) | 10.95 | 38.05 |
| 8 | Compound (e) | 10.77 | 37.63 |
| 9 | Compound (f) | 11.33 | 38.05 |
| Comp. I | CME | 12.11 | 35.22 |
| Comp. II | Aminol A-15 | 11.77 | 37.29 |
| Comp. III | Compound (g) | 13.95 | 36.74 |

These results show clearly that the levelling agents in accordance with the invention, exhibit not only better levelling effect (LE), but also better colouring intensity on healthy hear (ΔE HH) than the levelling agents of the comparative experiments.
The combination of levelling agents according to the invention was found to be particularly useful (**Example 6**).
Modifications, which do not affect, alter, change or modify the essential aspects of the compositions described, are included within the scope of the present invention.

## Claims

**1.** A dyeing composition comprising

a) a dyestuff; and

b) one or more compounds of the following formulae (I) and (II):

I 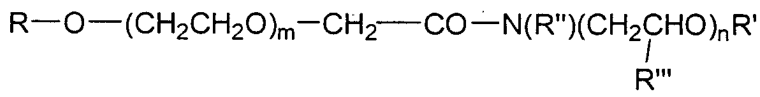

II 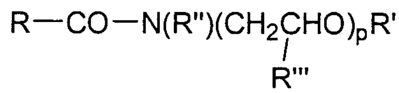

wherein

R is an alkyl group or alkenyl group having 6 to 24 carbon atoms;

R', R'' and R''' are independently a hydrogen atom or an alkyl group having 1 to 5 carbon atoms which is optionally hydroxylated;

m has a value in the range of 4 to 10;

n has a value in the range of 0 to 10; and

p has a value in the range of 1 to 10, with the proviso that when p = 1, at least one of R', R'' and R' ' ' is not a hydrogen atom.

**2.** A composition according to claim 1, wherein R in formula I or II is an alkyl group or alkenyl group having 12 to 24 carbon atoms.

**3.** A composition according to claims 1 or 2, wherein R', R' ' and R' ' ' in formula I or II are independently a hydrogen atom, a methyl group, an ethyl group or an hydroxyethyl group.

**4.** A composition according to any of the previous claims, wherein m in formula I has a value in the range of 4 to 8.

**5.** A composition according to any of the previous claims, wherein n in formula I has a value in the range of 0 to 5.

**6.** A composition according to any of the previous claims, wherein the sum of m and n in formula I is in the range of 5 to 9.

**7.** A composition according to claim 6, wherein m has a value in the range of 4 to 8, n has a value of 1, R is an alkyl group or alkenyl group having 12 to 18 carbon atoms; R' and R' ' represent a hydrogen atom, and R' ' ' represents a hydrogen atom or a methyl group.

**8.** A composition according to any of claims 1 to 3, wherein p in formula II has a value in the range of 3 to 5.

**9.** A composition according to any of claims 1 to 3 and 8 wherein the compound of formula II is one of the following formula III:

III 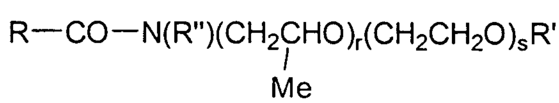

wherein r has a value in the range of 1 to 3, s has a value in the range of 1 to 3, and Me means a methyl group.

**10.** A composition according to claim 9, wherein R is an alkyl or alkenyl group having 12 to 24 carbon atoms, R' and R' ' represent a hydrogen atom, r is 1, and s has a value in the range of 1 to 3.

**11.** A composition according to claims 1 to 3, wherein p in formula II has a value of 1 or 2, and R ' ' represents a methyl group, ethyl group, or hydroxyethyl group.

**12.** A composition according to any of the preceding claims, further comprising ethoxylated products derived from polyhydric alcohols.

**13.** A composition according to claim 12, wherein the degree of ethoxylation of said ethoxylated products is more than 2.

**14.** A composition according to claims 12 or 13, wherein the polyhydric alcohol is glycerine.

**15.** A composition according to any of the preceding claims, further comprising an oxidizing agent.

**16.** A composition according to any of the preceding claims, further comprising an oxidation dye.

**17.** A composition according to any of the preceding claims, wherein one or more compounds of formula (I) or (II) are present in an amount of 0.05 to 25 wt.-% with respect to the total weight of the composition.

**18.** A method for dyeing keratinous fibres comprising applying an effective amount of at least one composition according to claims 1 to 17 to keratinous fibres.

**19.** A method for dyeing keratinous fibres according to claim 18, wherein said keratinous fibres are human hair.

**20.** The use of a compound of formula (I) or (II) as defined in any of claims 1 to 11 as levelling agent in compositions for dyeing keratinous fibres.

European Patent Office

# EUROPEAN SEARCH REPORT

Application Number
EP 02 00 4307

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DE 197 01 422 C (GOLDWELL GMBH) 5 March 1998 (1998-03-05) * page 2, line 5 - line 25; examples * | 1-20 | A61K7/13 |
| X | DD 271 219 A (MILTITZ CHEM WERK) 30 August 1989 (1989-08-30) * the whole document * | 1-20 | |
| A | US 6 130 252 A (SIEDMAN MICHAEL A ET AL) 10 October 2000 (2000-10-10) * column 5, line 65 - column 6, line 17 * | | |
| X | DE 196 25 738 A (BEIERSDORF AG) 2 January 1998 (1998-01-02) * examples 12,13,15 * | 1-19 | |
| X | WO 99 36047 A (SQUIBB BRISTOL MYERS CO) 22 July 1999 (1999-07-22) * example 10 * | 1-19 | |

TECHNICAL FIELDS SEARCHED (Int.Cl.7)

A61K

The present search report has been drawn up for all claims

| Place of search | Date of completion of the search | Examiner |
|---|---|---|
| MUNICH | 11 July 2002 | Simon, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 00 4307

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2002

| Patent document cited in search report | | Publication date | Patent family member(s) | | | Publication date |
|---|---|---|---|---|---|---|
| DE 19701422 | C | 05-03-1998 | DE | 19701422 | C1 | 05-03-1998 |
| DD 271219 | A | 30-08-1989 | DD | 271219 | A3 | 30-08-1989 |
| US 6130252 | A | 10-10-2000 | US | 5681859 | A | 28-10-1997 |
| | | | AT | 404310 | B | 27-10-1998 |
| | | | AT | 186894 | A | 15-03-1998 |
| | | | AU | 687076 | B2 | 19-02-1998 |
| | | | AU | 7158794 | A | 13-04-1995 |
| | | | BE | 1009621 | A5 | 03-06-1997 |
| | | | CA | 2130375 | A1 | 02-04-1995 |
| | | | CH | 688603 | A5 | 15-12-1997 |
| | | | CN | 1103755 | A | 21-06-1995 |
| | | | DE | 4435114 | A1 | 06-04-1995 |
| | | | DK | 76296 | A | 08-07-1996 |
| | | | DK | 112894 | A | 02-04-1995 |
| | | | ES | 2092955 | A1 | 01-12-1996 |
| | | | FR | 2710496 | A1 | 07-04-1995 |
| | | | IL | 110779 | A | 28-10-1999 |
| | | | IT | RM940587 | A1 | 03-04-1995 |
| | | | NL | 9401569 | A | 01-05-1995 |
| | | | RU | 2127518 | C1 | 20-03-1999 |
| | | | SE | 9403056 | A | 02-04-1995 |
| | | | US | 5614558 | A | 25-03-1997 |
| | | | US | 5998475 | A | 07-12-1999 |
| | | | ZA | 9406377 | A | 31-03-1995 |
| DE 19625738 | A | 02-01-1998 | DE | 19625738 | A1 | 02-01-1998 |
| | | | WO | 9749377 | A1 | 31-12-1997 |
| | | | EP | 0912159 | A1 | 06-05-1999 |
| | | | JP | 2000512647 | T | 26-09-2000 |
| WO 9936047 | A | 22-07-1999 | US | 6156076 | A | 05-12-2000 |
| | | | AU | 1633799 | A | 02-08-1999 |
| | | | BR | 9814008 | A | 10-10-2000 |
| | | | CA | 2317487 | A1 | 22-07-1999 |
| | | | CN | 1285736 | T | 28-02-2001 |
| | | | EP | 1047375 | A1 | 02-11-2000 |
| | | | JP | 2002509099 | T | 26-03-2002 |
| | | | WO | 9936047 | A1 | 22-07-1999 |

EPO FORM P0459

For more details about this annex : see Official Journal of the European Patent Office, No. 12/82